# EUROPEAN PATENT APPLICATION

(11) **EP 3 643 225 A1**
(43) Date of publication of application: **29.04.2020**
(21) Application number: 18820581.9
(22) Date of filing: 25.06.2018
(51) Int. Cl.: A61B 5/021, A61B 5/11, A61B 5/0408, A61B 5/00

(54) **BLOOD PRESSURE MEASUREMENT DEVICE**

(30) Priority: 23.06.2017 KR 20170079573
(71) Applicant: InBody Co., Ltd., Seoul 06106 (KR)
(72) Inventor: CHA, Ki Chul, Seoul 06544 (KR)
(74) Representative: V.O.
(86) International application number: PCT/KR2018/007162
(87) International publication number: WO 2018/236198

(57) **Abstract**

Provided is a blood pressure measurement device comprising: a chair unit provided such that a person for whom blood pressure is to be measured sits thereon; and a cuff part coupled to one side of the chair unit so as to move according to a preset direction and angle, and covering a person's arm for which blood pressure is to be measured.

## Description

### Technical Field

Example embodiments relate to a blood pressure measurement device, and more particularly, to a device on which a person may lean his/her body to measure a blood pressure of the person.

### Background Art

A blood pressure is used as a measure of a health condition of an individual. A blood pressure measurement device configured to measure a blood pressure is used generally in medical institutions and at homes. To measure a blood pressure, a pressure is applied to an arm of a subject to block a blood flow in a portion by which arterial blood passes and then the pressure applied is being slowly reduced. After this, a blood pressure is measured at a point in time at which an initial pulse sound is heard. The blood pressure measured at such point is referred to as a systolic blood pressure. A blood pressure is then measured at a point in time at which the pulse sound disappears. The pressure measured at such point is referred to as a diastolic blood pressure. An automatic blood pressure measurement device is configured to detect a waveform of a pressure that is measured while a pressure is being applied to an arm of a subject, and calculate a blood pressure therefrom.

When a blood pressure of a subject is measured while the subject sits, a blood pressure of the subject may change by a position of an arm of the subject. Thus, to measure an accurate blood pressure of the subject, it may be desirable that a height of a heart of the subject and a height of the arm of the subject from which a blood pressure is to be measured are in alignment with each other.

In general, when where the arm, a measurement target from which a blood pressure of the subject is to be measured, is positioned is lower than where the heart is positioned, a relatively higher blood pressure may be measured. In contrast, when where the arm is positioned is higher than where the heart is positioned, a relatively lower blood pressure may be measured. Thus, it may be desirable that the height of the arm and the height of the heart correspond to each other for measuring a blood pressure of the subject.

In many cases, a patient may use an automatic blood pressure measurement device to measure a blood pressure by himself/herself without an assistant who helps the patient use the device. However, the patient may not be fully aware of a measurement position and a correct posture when measuring a blood pressure, and even if he/she is, placing an arm of the patient at a right position may not be an easy task.

### Disclosure

### Technical Solutions

An aspect of the present disclosure provides an integral chair-cuff blood pressure measurement device that enables a user to sit in an accurate measurement posture required for measuring a blood pressure of the user without an assistance from a professional.

According to an example embodiment, there is provided a blood pressure measurement device including a chair provided such that a subject from which a blood pressure is to be measured is able to sit thereon, and a cuff connected to one side of the chair, and configured to accommodate an arm of the subject and cover the arm of the subject while being movable in a direction and at an angle that allow the arm of the subject to be in a predetermined posture for measuring the blood pressure of the subject.

For example, the cuff may be flexibly movable in all directions, and wound around a measurement portion, for example, an upper arm, from which the blood pressure is to be measured, in a vertical direction.

The blood pressure measurement device may further include a posture detector configured to detect a posture of the subject. The blood pressure measurement device may further include a suitability determiner configured to determine measurement suitability based on the detected posture.

The blood pressure measurement device may further include a cuff adjuster configured to adjust the cuff based on the measurement suitability.

The posture detector may be configured to detect a height of the arm of the subject and a height of a heart of the subject.

When the height of the arm and the height of the heart of the subject correspond to each other, the suitability determiner may be configured to determine the measurement suitability. When the height of the arm and the height of the heart do not correspond to each other, the suitability determiner may be configured to control the cuff adjuster to adjust a height of the cuff such that the height of the arm and the height of the heart correspond to each other.

The posture detector may be configured to detect the height of the arm and the height of the heart of the subject through image recognition of a body part of the subject.

The posture detector may be configured to detect the height of the arm using one of an acceleration sensor and a gyro sensor that is connected to the cuff, and detect the height of the heart of the subject using one of a capacitance sensor and an electrocardiogram (ECG) sensor that is connected to one side of a backrest of the chair.

The posture detector may be configured to detect a contact between a back of the subject and the backrest formed in the chair.

When the back of the subject and the backrest formed in the chair are in contact with each other, the suitability determiner may be configured to determine the measurement suitability. When the back of the subject and the backrest formed in the chair are separate from each other, the suitability determiner may be configured to generate a notification such that the subject recognizes that the back of the subject and the backrest formed in the chair are separate from each other.

The posture detector may be configured to detect the contact using one of a capacitance sensor and an ECG sensor that is connected to one side of the backrest of the chair.

The posture detector may be configured to detect an angle of the arm of the subject.

When the angle of the arm of the subject is parallel to the ground, the suitability determiner may be configured to determine the measurement suitability. When the angle of the arm of the subject is not parallel to the ground, , the suitability determiner may be configured to control the cuff adjuster to adjust an angle of the cuff such that the angle of the arm is parallel to the ground.

The cuff may be a bracelet-type cuff that is separable and connectable in a vertical or horizontal direction based on a hinge formed in the cuff.

The direction and the angle of the cuff may be set such that the arm of the subject is covered vertically.

According to another example embodiment, there is provided a blood pressure measurement device including a first sensor configured to measure a height of a subject, an armrest configured to fix a height of an arm of the subject for measuring a blood pressure of the subject, a driver configured to provide an assistance force that allows the armrest to move vertically in accordance with the measured height of the subject, a cuff including a movable member configured to move in a direction and at an angle that allows the arm of the subject to be in a predetermined posture, and connected to one side of the armrest to accommodate and cover the arm of the subject for measuring the blood pressure, a sensor portion configured to sense an angle formed by the cuff with respect to the ground, and a controller configured to control an angular movement of the movable member based on the sensed angle.

The controller may be configured to control the angular movement of the movable member such that the angle formed by the cuff with respect to the ground is in a range based on 10.7 degrees (°).

The blood pressure measurement device may further include a second sensor configured to detect a contact between the subject and the blood pressure measurement device. The cuff may further include an elbow groove on which an elbow of the subject is to be seated. When the contact is detected by the second sensor and the elbow is seated on the elbow groove, the cuff may be configured to apply a pressure to the arm of the subject.

The driver may be configured to provide an assistance force that allows the armrest to rotatably move on a predetermined plane.

According to still another example embodiment, there is provided a blood pressure measurement device including a chair configured to provide a subject with a space to sit for measuring a blood pressure of the subject, a hollow portion formed on one side of a backrest included in the chair and including a plurality of holes to adjust a height of an armrest, the armrest including a connecting pin and configured to fix an arm of the subject by connecting the connecting pin to one of the holes of the hollow portion, a cuff connected to one side of the chair and configured to accommodate the arm of the subject and cover the arm of the subject while being movable in a direction and at an angle that allow the arm of the subject to be in a predetermined posture for measuring the blood pressure of the subject, a sensor portion configured to sense an angle formed by the cuff with respect to the ground, and a controller configured to control an angular movement of the cuff based on the sensed angle.

The controller may be configured to control the angular movement of the cuff such that the sensed angle is in a preset angle range.

The armrest may be configured to connect the connecting pin to one of the holes of the hollow portion such that a height of the arm of the subject and a height of a heart of the subject correspond to each other.

The cuff may be a bracelet-type cuff that is separable and connectable in a vertical or horizontal direction based on a hinge to be connected to the armrest.

### Brief Description of Drawings

FIG. 1 is a perspective view of a blood pressure measurement device according to an example embodiment.
FIG. 2 is a first side view of a blood pressure measurement device according to an example embodiment.
FIG. 3 is a second side view of a blood pressure measurement device according to an example embodiment.
FIG. 4 is a diagram illustrating an example of a determination of a position of a heart through image recognition according to an example embodiment.
FIG. 5 is a side view of a blood pressure measurement device according to another example embodiment.
FIG. 6a is a perspective view of a blood pressure measurement device according to still another example embodiment.
FIG. 6b is a plane view of the blood pressure measurement device of FIG. 6a.

### Best Mode for Carrying Out the Invention

Hereinafter, example embodiments will be described in detail with reference to the accompanying drawings. Regarding the reference numerals assigned to the elements in the drawings, it should be noted that the same elements will be designated by the same reference numerals, wherever possible, even though they are shown in different drawings.

The following detailed description is provided to assist the reader in gaining a comprehensive understanding of the methods, apparatuses, and/or systems described herein. However, various changes, modifications, and equivalents of the methods, apparatuses, and/or systems described herein will be apparent after an understanding of the disclosure of this application. For example, the sequences of operations described herein are merely examples, and are not limited to those set forth herein, but may be changed as will be apparent after an understanding of the disclosure of this application, with the exception of operations necessarily occurring in a certain order.

Although terms such as "first," "second," and "third" may be used herein to describe various members, components, regions, layers, or sections, these members, components, regions, layers, or sections are not to be limited by these terms. Rather, these terms are only used to distinguish one member, component, region, layer, or section from another member, component, region, layer, or section. Thus, a first member, component, region, layer, or section referred to in examples described herein may also be referred to as a second member, component, region, layer, or section without departing from the teachings of the examples.

Throughout the specification, when a component is described as being "connected to," or "coupled to" another component, it may be directly "connected to," or "coupled to" the other component, or there may be one or more other components intervening therebetween. In contrast, when an element is described as being "directly connected to," or "directly coupled to" another element, there can be no other elements intervening therebetween. Likewise, similar expressions, for example, "between" and "immediately between," and "adjacent to" and "immediately adjacent to," are also to be construed in the same way. As used herein, the term "and/or" includes any one and any combination of any two or more of the associated listed items.

The terminology used herein is for describing various examples only and is not to be used to limit the disclosure. The articles "a," "an," and "the" are intended to include the plural forms as well, unless the context clearly indicates otherwise. The terms "comprises," "includes," and "has" specify the presence of stated features, numbers, operations, members, elements, and/or combinations thereof, but do not preclude the presence or addition of one or more other features, numbers, operations, members, elements, and/or combinations thereof.

The features described herein may be embodied in different forms and are not to be construed as being limited to the examples described herein. Rather, the examples described herein have been provided merely to illustrate some of the many possible ways of implementing the methods, apparatuses, and/or systems described herein that will be apparent after an understanding of the disclosure of this application.

Unless otherwise defined, all terms, including technical and scientific terms, used herein have the same meaning as commonly understood by one of ordinary skill in the art to which this disclosure pertains and based on an understanding of the disclosure of the present application. Terms, such as those defined in commonly used dictionaries, are to be interpreted as having a meaning that is consistent with their meaning in the context of the relevant art and the disclosure of the present application and are not to be interpreted in an idealized or overly formal sense unless expressly so defined herein.

Also, in the description of example embodiments, detailed description of structures or functions that are thereby known after an understanding of the disclosure of the present application will be omitted when it is deemed that such description will cause ambiguous interpretation of the example embodiments.

Hereinafter, a blood pressure measurement device in which a cuff and an armrest are combined with a chair will be described in detail with reference to the accompanying drawings according to example embodiments. However, the blood pressure measurement device may also be implemented only with an armrest embodied only by a combination of a cuff and an arm support. For example, the blood pressure measurement device that enables a user to measure a blood pressure by putting his/her arm into the cuff attached to an outer wall while the user is standing without a chair may also be in the scope of the present disclosure. In addition, the blood pressure measurement device may also be implemented only with a cuff that is movable in a vertical direction and an arm support, and the blood pressure measurement device implemented as described in the foregoing may also be in the scope of the present disclosure.

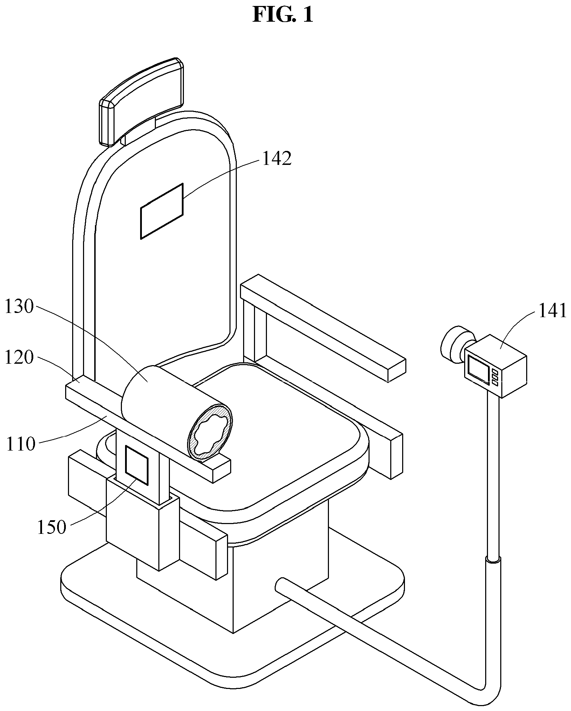

FIG. 1 is a perspective view of a blood pressure measurement device according to an example embodiment.

Referring to FIG. 1, a blood pressure measurement device according to an example embodiment includes a chair 110 and a cuff 130.

In addition, the blood pressure measurement device further includes an armrest 120, a posture detector 141 and 142, a suitability determiner (not shown), and a cuff adjuster 150.

The chair 110 is provided such that a subject, or a user from which a blood pressure is to be measured, is able to sit thereon for measuring a blood pressure of the subject. The chair 110 illustrated herein may be provided as one of various examples of application. Thus, other examples of application that may allow a user to lean or sit thereon for measuring a blood pressure of the user may also be applicable. For example, a massage chair, an office chair, a medical chair on which an outpatient visiting a dental clinic or other hospitals is able to sit, a car seat (e.g., a driver's seat and other passenger seats), a chair installed in an ambulance, and others may also be applicable. Thus, the chair 110 may not be limited to a certain type of chair.

The cuff 130 is connected to one side of the chair, and configured to cover or wrap an arm of the subject while being movable in a set direction and at a set angle to measure a blood pressure of the subject. For example, the cuff 130 is provided in an integral form to be integrated with the chair 110, and configured to move flexibly based on a direction and an angle that are set based on a detected posture and determined measurement suitability. That is, the cuff may move flexibly in all directions, and be wound vertically around a measurement portion (e.g., upper arm) from which the blood pressure is to be measured.

The cuff 130 may also be combined with the armrest 120 connected to one side of the chair and on which the subject comfortably places his/her arm.

The posture detector 141 and 142 is configured to detect a posture of the subject, and the suitability determiner is configured to determine measurement suitability based on the detected posture. The suitability determiner may include a general processor or a dedicated hardware.

The cuff adjuster 150 is configured to adjust the cuff 130 based on the determined measurement suitability. In detail, the cuff adjuster 150 may be selected from among various means that generate a physical movement to adjust a height of the cuff 130. For example, the various means may include a motor configured to provide a driving force, and other means including hydraulic and pneumatic means. The cuff adjuster 150 is controlled to reduce or minimize a difference between the height of the cuff 130 and a desirable height that is determined by the suitability determiner based on the posture of the subject recognized by the posture detector 141 and 142.

Here, a direction and an angle of the cuff may be set by the cuff adjuster 150 such that the arm of the subject is to be covered vertically. For example, the angle of the cuff may be readjusted such that a measurement portion of the arm is covered in a vertical direction even though a height of the arm changes.

Hereinafter, a structure by which the blood pressure measurement device measures an accurate blood pressure of a subject will be described in detail with reference to side views of the blood pressure measurement device.

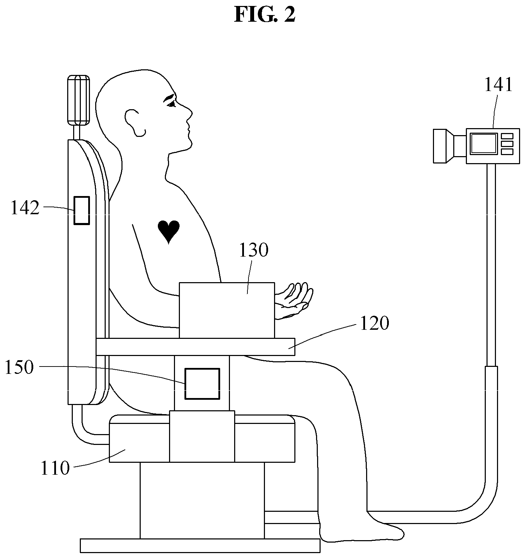

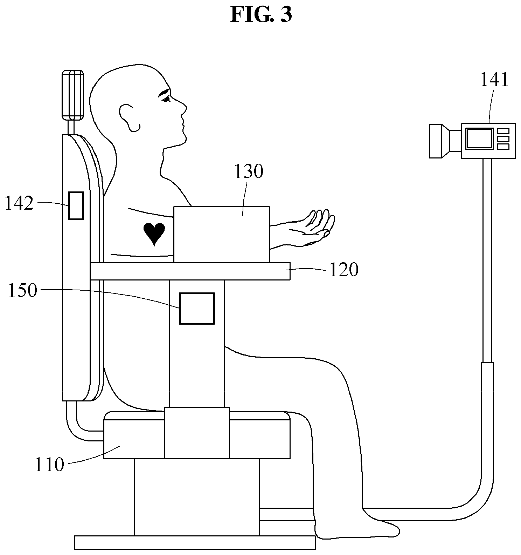

FIG. 2 is a first side view of a blood pressure measurement device according to an example embodiment. FIG. 3 is a second side view of a blood pressure measurement device according to an example embodiment.

To measure a blood pressure, a subject may place an arm of the subject inside the cuff 130 combined with the armrest 120 while the subject sits on the chair 110.

For example, the cuff 130 may be provided in a cylindrical form, and thus the subject may insert the arm into a measurement position through an entry of the cuff 130.

For another example, the cuff 130 may be provided in a bracelet type that is separable and connectable in a vertical or horizontal direction based on a hinge formed in the cuff 130. In this example, in a case of the cuff 130 that is separable and connectable in the vertical direction, the subject may place the arm at the measurement position by placing the arm on an upper surface of a separated lower portion of the cuff 130 and closing a separated upper portion of the cuff 130 to fasten it to the lower separated portion.

When the subject places the arm inside the cuff 130, the posture detector 141 and 142 may detect a height of the arm of the subject and a height of a heart of the subject.

For example, the posture detector 141 may detect the height of the arm and the height of the heart through image recognition of a body part of the subject. Referring to the first side view of FIG. 2, a height of a heart of a user and a height of an arm of the user do not correspond to each other. However, referring to the second side view of FIG. 3, as a height of the armrest 120 is adjusted, the height of the heart of the user and the height of the arm of the user correspond to each other.

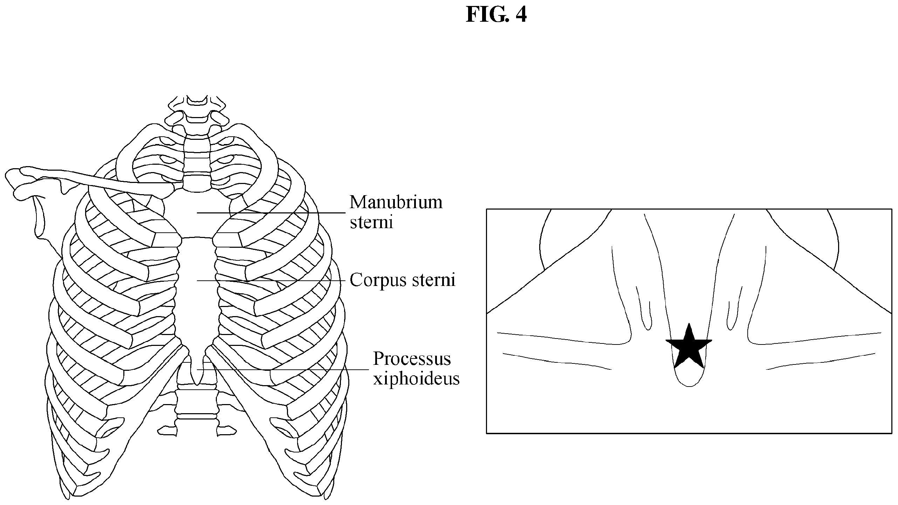

FIG. 4 is a diagram illustrating an example of a determination of a position of a heart through image recognition according to an example embodiment.

Referring to FIG. 4, the posture detector 141 may be a capturing device configured to capture an image, and may analyze a captured image of a subject to detect a height of an arm of the subject and a height of a heart of the subject.

For example, a position of the heart may be determined as midsternal, which is a position in the middle between jugular notch and xiphoid process (which is a small cartilaginous tissue positioned at a lower tip of sternum).

In detail, a position of the jugular notch and a position of the xiphoid process may be indicated on a top of the subject, and then the position of the heart may be set as a middle point between the jugular notch and the xiphoid process.

In addition, the posture detector 142 may detect the height of the arm using one of an acceleration sensor and a gyro sensor that are connected to the armrest 120, and detect the height of the heart using one of a capacitance sensor, a microwave antennal, and an electrocardiogram (ECG) sensor that are connected to one side of a backrest of the chair.

The suitability determiner may determine measurement suitability when the height of the arm of the subject corresponds to the height of the heart and otherwise, adjust the height such that the height of the arm and the height of the heart corresponds to each other.

For example, when the height of the arm of the subject corresponds to the height of the heart of the subject, the suitability determiner may determine the measurement suitability. However, when the height of the arm and the height of the heart do not correspond to each other as illustrated in FIG. 2, the suitability determiner may control the cuff adjuster 150 to adjust the height of the armrest 120 such that the height of the arm and the height of the heart correspond to each other.

As the height of the armrest is adjusted automatically, the height of the arm of the subject and the height of the heart of the subject may become the same, enabling more accurate measurement of a blood pressure of the subject.

In addition to a position of the arm, a sitting posture of the subject may also affect a result of the measurement of a blood pressure of the subject. Thus, it may be desirable for the subject to attach a back of the subject to the backrest of the chair 110 without bending a body of the subject.

According to an example embodiment, the blood pressure measurement device may determine the measurement suitability when the back of the subject is in contact with the backrest without the body of the subject being bent. When the measurement suitability is not determined, the blood pressure measurement device may generate a notification.

The posture detector 142 may detect a contact between the back of the subject and the backrest formed in the chair.

For example, the posture detector 142 may detect the contact using one of a capacitance sensor and an ECG sensor that is connected to one side of the backrest.

In this example, when the back of the subject is in contact with the backrest formed in the chair, the suitability determiner may determine the measurement suitability. When the back of the subject is separate from the chair, the suitability determiner may generate a notification such that the subject recognizes that back of the subject is separate from the chair.

In addition to the position of the arm and the sitting posture, an angle of the arm of the subject may also affect a result of the measurement of a blood pressure of the subject. Thus, it may be desirable that the angle of the arm is maintained being parallel to the ground for the measurement of the blood pressure.

When the arm is parallel to the ground, the blood pressure measurement device may determine the measurement suitability and otherwise, adjust the angle of the arm.

The posture detector may detect an angle of the arm of the subject. When the angle of the arm is parallel to the ground, the suitability determiner may determine the measurement suitability. However, when the angle of the arm is not parallel to the ground, the suitability determiner may control the cuff adjuster 150 to adjust an angle of the cuff 130 such that the angle of the arm becomes parallel to the ground.

As described above, it is possible to measure an accurate blood pressure by detecting a posture of a subject and correcting the posture of the subject to be suitable for the measurement. Thus, without assistance from a professional, the blood pressure measurement device, which is a chair-type blood pressure measurer, for example, may enable a sitting posture of the subject to be desirable for the measurement of a blood pressure. Thus, a measurement portion of the subject from which the blood pressure is to be measured may be maintained in a desirable state and/or posture without the subject intentionally correcting his/her posture. Thus, it is possible to improve accuracy in measurement of a blood pressure, and reproducibility and convenience.

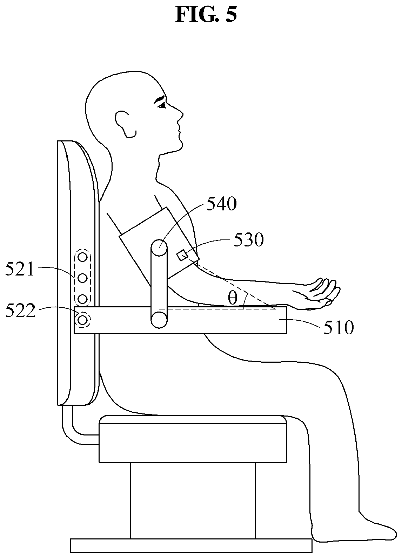

FIG. 5 is a side view of a blood pressure measurement device according to another example embodiment. Referring to FIG. 5, a blood pressure measurement device includes an armrest 510 including a connecting pin 522, a hollow portion 521, a sensor 530, and a cuff 540. The blood pressure measurement device includes a chair that provides a subject with a space to sit thereon for measuring a blood pressure of the subject. The hollow portion 521 is formed on one side of a backrest included in the chair, and includes a plurality of holes to adjust a height of the armrest. Using the armrest 510 and the hollow portion 521, it is possible to adjust a measurement height such that a height of an arm of the subject corresponds to a height of a heart of the subject, and provide a more accurate result of measuring a blood pressure of the subject.

The armrest 510 includes the connecting pin 522 to be inserted into one of the holes of the hollow portion 521. The armrest 510 may set or fix a height of the arm of the subject by connecting the connecting pin 522 to one of the holes of the hollow portion 521. Thus, the armrest 510 may allow the height of the arm of the subject to correspond to the height of the heart of the subject.

In addition, the cuff 540 provided to measure the blood pressure is connected to one side of the armrest 510. In detail, the cuff 540 and the armrest 510 may be connected by a first hinge and a second hinge each having a degree of freedom (DoF) in a preset direction. The cuff 540 may accommodate the arm of the subject and move in a direction and at an angle such that the arm of the subject is in a predetermined posture. The cuff 540 may cover or wrap the arm of the subject to measure a blood pressure of the subject. The cuff 540 may be provided in a bracelet type that is separable and connectable in a vertical or horizontal direction based on the first hinge connected to the armrest 510.

The sensor 530 may sense an angle of the cuff that is formed with respect to the ground. The sensor 530 may output angle data corresponding to the sensed angle. The sensor 530 may be embodied as an inertial measurement unit (IMU), for example. The IMU may be a gyro sensor configured to measure a rotational inertia.

Although not illustrated in FIG. 5, the first hinge and the second hinge connected to the cuff 540 and the armrest 510 may further include a controller. The controller may control an angular movement of the cuff 540 based on the sensed angle. The controller may control the angular movement of the cuff 540 such that the sensed angle is in a preset angle range.

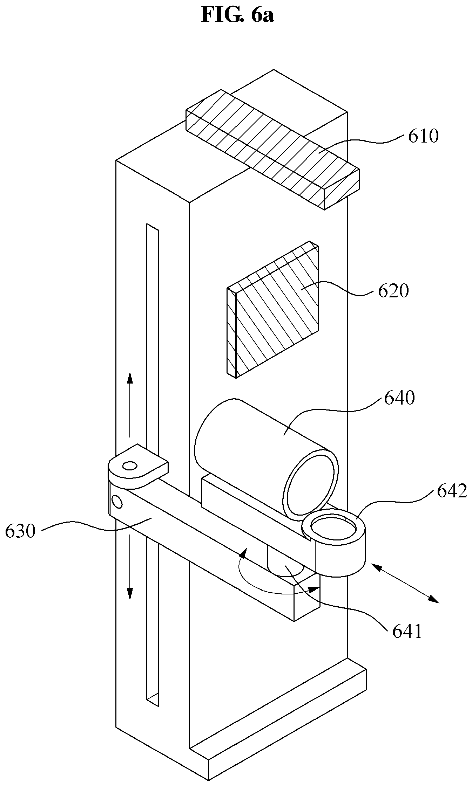

FIG. 6a is a perspective view of a blood pressure measurement device according to still another example embodiment. Referring to FIG. 6a, a blood pressure measurement device includes a first sensor 610,m a second sensor 620, an armrest 630, and a cuff 640. In detail, the cuff 640 includes a movable member 641 and an elbow groove 642. The first sensor 610 is configured to measure a height of a subject. The second sensor 620 is configured to sense a contact between the subject and the blood pressure measurement device. For example, the second sensor 620 may detect whether a back of the subject comes into contact with the blood pressure measurement device. The armrest 630 is configured to set or fix a height of an arm of the subject to enable measurement of a blood pressure of the subject. Although not illustrated in FIG. 6a, the blood pressure measurement device further includes a driver. The driver may be a mechanical device configured to generate power using a preset energy source. The driver is configured to provide an assistance force that enables the armrest 630 to move in a vertical direction in accordance with the height of the subject measured by the first sensor 610.

The cuff 640 is connected to one side of the armrest 630, and configured to accommodate the arm of the subject and cover the arm to measure a blood pressure of the subject. In addition, the cuff 640 includes the movable member 641 that moves in a direction and at an angle such that the arm of the subject is in a predetermined posture. The movable member 641 may provide a DoF that allows the cuff 640 to move forward or backward or rotate within a preset plane in accordance with a size and a length of the arm of the subject.

The cuff 640 further includes the elbow groove 642 that allows an elbow of the subject to be seated thereon. To the cuff 640, a sensor configured to sense an angle of the cuff 640 formed with respect to the ground may be connected. In addition, the blood pressure measurement device may control or restrict an angular movement of the movable member 641 based on the sensed angle. For example, a controller may control the angular movement of the movable member 641 such that the cuff 640 has an angle within a range based on 10.7 degrees (°) formed with respect to the ground.

According to this example embodiment, when the second sensor 620 senses the contact between the subject and the blood pressure measurement device and the elbow of the subject is seated on the elbow groove 642, the cuff 640 may press, or apply a pressure to, the arm of the subject.

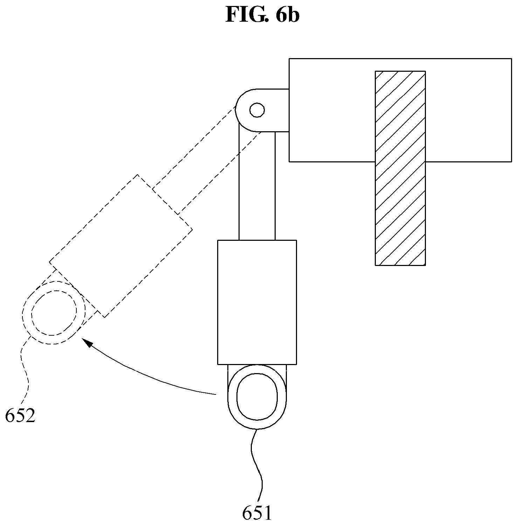

FIG. 6b is a plane view of the blood pressure measurement device of FIG. 6a. The driver may provide an assistance force that enables the armrest to rotatably move from a first position 651 to a second position 652. In detail, the driver may provide an assistance force that enables the armrest 630 to rotatably move within a preset angle in a predefined plane. Thus, according to this example embodiment, the blood pressure measurement device may provide convenience to users with various physical body sizes and measured postures.

The units described herein may be implemented using hardware components and software components. For example, the hardware components may include microphones, amplifiers, band-pass filters, audio to digital convertors, non-transitory computer memory and processing devices. A processing device may be implemented using one or more general-purpose or special purpose computers, such as, for example, a processor, a controller and an arithmetic logic unit (ALU), a digital signal processor, a microcomputer, a field programmable gate array (FPGA), a programmable logic unit (PLU), a microprocessor or any other device capable of responding to and executing instructions in a defined manner. The processing device may run an operating system (OS) and one or more software applications that run on the OS. The processing device also may access, store, manipulate, process, and create data in response to execution of the software. For purpose of simplicity, the description of a processing device is used as singular; however, one skilled in the art will appreciated that a processing device may include multiple processing elements and multiple types of processing elements. For example, a processing device may include multiple processors or a processor and a controller. In addition, different processing configurations are possible, such a parallel processors.

The software may include a computer program, a piece of code, an instruction, or some combination thereof, to independently or collectively instruct or configure the processing device to operate as desired. Software and data may be embodied permanently or temporarily in any type of machine, component, physical or virtual equipment, computer storage medium or device, or in a propagated signal wave capable of providing instructions or data to or being interpreted by the processing device. The software also may be distributed over network coupled computer systems so that the software is stored and executed in a distributed fashion. The software and data may be stored by one or more non-transitory computer readable recording mediums. The non-transitory computer readable recording medium may include any data storage device that can store data which can be thereafter read by a computer system or processing device.

The methods according to the above-described example embodiments may be recorded in non-transitory computer-readable media including program instructions to implement various operations of the above-described example embodiments. The media may also include, alone or in combination with the program instructions, data files, data structures, and the like. The program instructions recorded on the media may be those specially designed and constructed for the purposes of example embodiments, or they may be of the kind well-known and available to those having skill in the computer software arts. Examples of non-transitory computer-readable media include magnetic media such as hard disks, floppy disks, and magnetic tape; optical media such as CD-ROM discs, DVDs, and/or Blue-ray discs; magneto-optical media such as optical discs; and hardware devices that are specially configured to store and perform program instructions, such as read-only memory (ROM), random access memory (RAM), flash memory (e.g., USB flash drives, memory cards, memory sticks, etc.), and the like. Examples of program instructions include both machine code, such as produced by a compiler, and files containing higher level code that may be executed by the computer using an interpreter. The above-described devices may be configured to act as one or more software modules in order to perform the operations of the above-described example embodiments, or vice versa.

While this disclosure includes specific examples, it will be apparent to one of ordinary skill in the art that various changes in form and details may be made in these examples without departing from the spirit and scope of the claims and their equivalents. The examples described herein are to be considered in a descriptive sense only, and not for purposes of limitation. Descriptions of features or aspects in each example are to be considered as being applicable to similar features or aspects in other examples. Suitable results may be achieved if the described techniques are performed in a different order, and/or if components in a described system, architecture, device, or circuit are combined in a different manner and/or replaced or supplemented by other components or their equivalents.

Therefore, the scope of the disclosure is defined not by the detailed description, but by the claims and their equivalents, and all variations within the scope of the claims and their equivalents are to be construed as being included in the disclosure.

## Claims

1. A blood pressure measurement device comprising:
a chair provided such that a subject from which a blood pressure is to be measured is able to sit thereon; and
a cuff connected to one side of the chair, and configured to accommodate an arm of the subject and cover the arm of the subject while being movable in a direction and at an angle that allow the arm of the subject to be in a predetermined posture for measuring the blood pressure of the subject.

2. The blood pressure measurement device of claim 1, further comprising:
a posture detector configured to detect a posture of the subject.

3. The blood pressure measurement device of claim 2, further comprising:
a suitability determiner configured to determine measurement suitability based on the detected posture.

4. The blood pressure measurement device of claim 3, further comprising:
a cuff adjuster configured to adjust the cuff based on the measurement sui tabili ty.

5. The blood pressure measurement device of claim 2, wherein the posture detector is configured to:
detect a height of the arm of the subject and a height of a heart of the subject.

6. The blood pressure measurement device of claim 4, wherein the suitability determiner is configured to:
when a height of the arm and a height of a heart of the subject correspond to each other, determine the measurement suitability; and
when the height of the arm and the height of the heart do not correspond to each other, control the cuff adjuster to adjust a height of the cuff such that the height of the arm and the height of the heart correspond to each other.

7. The blood pressure measurement device of claim 2, wherein the posture detector is configured to:
detect a height of the arm and a height of a heart of the subject through image recognition of a body part of the subject.

8. The blood pressure measurement device of claim 2, wherein the posture detector is configured to:
detect a height of the arm using one of an acceleration sensor and a gyro sensor that is connected to the cuff; and
detect a height of a heart of the subject using one of a capacitance sensor and an electrocardiogram (ECG) sensor that is connected to one side of a backrest of the chair.

9. The blood pressure measurement device of claim 1, wherein the posture detector is configured to:
detect a contact between a back of the subject and a backrest formed in the chair.

10. The blood pressure measurement device of claim 3, wherein the suitability determiner is configured to:
when a back of the subject and a backrest formed in the chair are in contact with each other, determine the measurement suitability; and
when the back of the subject and the chair are separate from each other, generate a notification such that the subject recognizes that the back of the subject and the chair are separate from each other.

11. The blood pressure measurement device of claim 2, wherein the posture detector is configured to:
detect the contact using one of a capacitance sensor and an ECG sensor that is connected to one side of the backrest of the chair.

12. The blood pressure measurement device of claim 2, wherein the posture detector is configured to:
detect an angle of the arm of the subject.

13. The blood pressure measurement device of claim 4, wherein the suitability determiner is configured to:
when an angle of the arm of the subject is parallel to the ground, determine the measurement suitability; and
when the angle of the arm of the subject is not parallel to the ground, control the cuff adjuster to adjust an angle of the cuff such that the angle of the arm is parallel to the ground.

14. The blood pressure measurement device of claim 1, wherein the cuff is a bracelet-type cuff that is separable and connectable in a vertical or horizontal direction based on a hinge formed in the cuff.

15. The blood pressure measurement device of claim 1, wherein the direction and the angle of the cuff are set such that the arm of the subject is covered vertically.

16. A blood pressure measurement device comprising:
a first sensor configured to measure a height of a subject;
an armrest configured to fix a height of an arm of the subject for measuring a blood pressure of the subject;
a driver configured to provide an assistance force that allows the armrest to move vertically in accordance with the measured height of the subject;
a cuff including a movable member configured to move in a direction and at an angle that allows the arm of the subject to be in a predetermined posture, and connected to one side of the armrest to accommodate and cover the arm of the subject for measuring the blood pressure;
a sensor portion configured to sense an angle formed by the cuff with respect to the ground; and
a controller configured to control an angular movement of the movable member based on the sensed angle.

17. The blood pressure measurement device of claim 16, wherein the controller is configured to:
control the angular movement of the movable member such that the angle formed by the cuff with respect to the ground is in a range based on 10.7 degrees (°).

18. The blood pressure measurement device of claim 16, further comprising:
a second sensor configured to detect a contact between the subject and the blood pressure measurement device, and
the cuff further includes an elbow groove on which an elbow of the subject is to be seated,
wherein, when the contact is detected by the second sensor and the elbow is seated, the cuff is configured to apply a pressure to the arm of the subject.

19. The blood pressure measurement device of claim 16, wherein the driver is configured to provide an assistance force that allows the armrest to rotatably move on a predetermined plane.
